# EUROPEAN PATENT APPLICATION

(11) **EP 4 151 253 A1**
(43) Date of publication of application: **22.03.2023**
(21) Application number: 21804217.4
(22) Date of filing: 11.05.2021
(51) Int. Cl.: A61M 5/168

(54) **MULTIFUNCTIONAL DEVICE FOR CONTROLLING INTRAVENOUS DRIP INFUSION**

(30) Priority: 12.05.2020 ES 202030880 U
(71) Applicant: Urrea Rivas, Javier, 08224 Barcelona (ES)
(72) Inventor: Urrea Rivas, Javier, 08224 Barcelona (ES)
(74) Representative: Ungria López, Javier
(86) International application number: PCT/ES2021/070331
(87) International publication number: WO 2021/229127

(57) **Abstract**

The invention relates to a multifunctional device for controlling intravenous drip infusion, of the type comprising means for measuring the amount of liquid remaining in an individual drip infusion unit, and which are associated with a centralised monitoring module via a wireless data transmission module (2.5); comprising a housing in which are arranged: - an upper hook (1.5), - a semi-open lower hook (1.6) in which a pressure sensor (1.7) is integrated, - a camera (1.2), -a push button (1.8), associated with the microprocessor (2.3) and the camera (1.2) to connect the use of said camera (1.2), - a battery (2.2), and - a microprocessor (2.3).

## Description

### TECHNICAL SECTOR

The purpose of this utility model application is to register a multifunctional electronic device for centralised wireless monitoring of the unattended process of drip infusion for patients in medical centres. It forms part of a wireless connected system with a central monitoring module.

Unlike the rest of the different configurations of patented devices with a similar purpose, this one in particular among others, collaborates and seeks compliance with the Falsified Medicines Directive (Directive 2011/62/EU) which is mandatory in the European Union (EU) since 9 February 2019. It has been designed following the safety features marked in the Commission Delegated Regulation (EU) 2016/161. For this purpose, it consists of an optical display camera capable of reading the QR code or graphic semi-compressed reading information feature contained in each sales unit and/or product unit to be supplied. Cross-checking it, in this case, against the SEVEM (Spanish Medicine Verification System) database or server used for said control in each case. Thus verifying the authenticity of the medical product to be supplied.

At the same time, it is involved in the automated stock control process of hospital material and in the patient's history control record, in order to follow up and avoid possible allergic cases, among others. These can be avoided with useful instant information given to the medical staff prior to any action, based on data that is collected and cross-checked with the patient's history. In the wake of the recent pandemic, the concept of minimal nurse-patient contact is significant, as the number of room visits by the nurse is reduced.

### BACKGROUND OF THE INVENTION

Current drip supply detection devices measure the amount of liquid remaining in the individual unit by means of infrared, ultrasound or other measuring sensors, which provide information on the volume of liquid consumed or remaining, per unit of time. They are based on having access to information in a monitoring module, with a display or visual device that provides the method of interaction and one or more detection or measurement modules.

They use buzzer and/or light alarm systems to signal when the infusion unit is about to run out or abnormalities occur. A button can be provided in the measurement module for the patient to issue an early alarm to the medical service.

The control device may consist of a microprocessor, a power supply module, a data security module, a wireless data transmission module, a switch, a display, and a vibration and/or buzzer module. Furthermore, the measurement module may consist of a detection module, a power module, a wireless data transmission module, a switch and/or call button, a pressure sensor and a vibration and/or buzzer module.

The reference of previous applications are as follows:
1. Medical drip reminder system. Publication number CN105879153 (24/08/2016). Applicant: Xu Hui.
2. Medical drip detection reminding system. Publication number CN208989893 (18/06/2019). Applicant: Liu Meiying.
3. Automatic monitoring system device for unattended drip. Publication number: CN209108292 (16/07/2019). Applicant: EAST CHINA JIAOTONG UNIVERSITY.
4. Portable wireless medical drip monitoring system. Publication number: CN107583139 (16/01/2018). Applicant: Huzhou Huineng Electromechanical Technology Co., Ltd.
5. Drip detection system and method. Publication number: CN110025855 (19/07/2019). Applicant: Melten Corporation.
6. Infrared detection drip control system based on host control. Application number: CN104014017 (03/09/2014). Applicant: SUZHOU WAWU INTERNET OF THINGS TECHNOLOGY CO., LTD.
7. Ultrasonic detection drip monitoring system based on host control. Application number: CN104014027 (03/09/2014). Applicant: SUZHOU WAWU INTERNET OF THINGS TECHNOLOGY CO., LTD.
8. Pressure-detection-based drip control system with flow rate indication. Application number: CN104014055 (03/09/2014). Applicant: SUZHOU WAWU
9. Drip control system based on wireless host control and pressure detection. Application number: CN104014054 (03/09/2014). Applicant: SUZHOU WAWU INTERNET OF THINGS TECHNOLOGY CO., LTD.

### DESCRIPTION OF THE INVENTION

The present invention is a novel multifunctional device for monitoring unattended intravenous drip products. At the same time, it aims to comply with the regulations against counterfeit pharmaceuticals, by automatically checking each control serial number of each product to be supplied.

The invention pursues legality, practicality and efficiency in the daily life of our medical centres. This novel technological product presents, unlike its predecessors, a reader (e.g. a camera) for reading graphic codes of semi-compressed information, which can be QR codes. Thus, it allows to cross-check and confirm traceability before supply, according to regulations. This check is performed on the spot with the central European server, wherein it is verified whether the product is genuine. The code reader is integrated in the front portion of the device and is actuated by a reading push button of the same nature as a common camera. It also has a roughness in the lower portion which is opposite to the push button, which allows greater control of handling to the touch.

The code reader reads the reference code on the multifunctional measuring device. This information is encrypted and sent to the centralised monitoring module, which performs online checks through SEVEM access, verifying and deactivating said medicine in case of a positive result. Otherwise, an alarm is triggered in the multifunctional measuring device and in the central monitoring module.

Said code reader, which makes this configuration unique compared to its predecessors, can also be used in the automatic telecontrol. These other processes should be identifiable by means of graphic codes of semi-compressed information. Such as patient identification and its advantages can be.

The record associated with a hospital unit is performed with the help of a centralised monitoring and processing module. This module consists of a touch screen and can carry peripheral supports to aid interaction, such as keyboards. This central system processes the information received from the multifunctional measurement modules wirelessly, with data access and processing, and from the hospital's central server database. This latter use seeks the optimisation and automation of the processes and attached records, for a continuous improvement of the service and hospital administration.

In the event that the genuine sales unit and its respective control serial number represents a sales unit of more than one usable, we will use an individual record counter method per sales unit or case. This will allow us to have an exhaustive stock control, while reducing the possibility of a last possible malicious manipulation of the sales unit once it has been opened.

### BRIEF DESCRIPTION OF THE DRAWINGS

Two figures are attached for a better understanding of the invention:
Figure 1 shows the geometry of the invention in its two differentiated and constituent parts, and the internal detailed profiles of same.
Figure 2 shows an electronic diagram according to the present invention.

### PREFERRED EMBODIMENT OF THE INVENTION

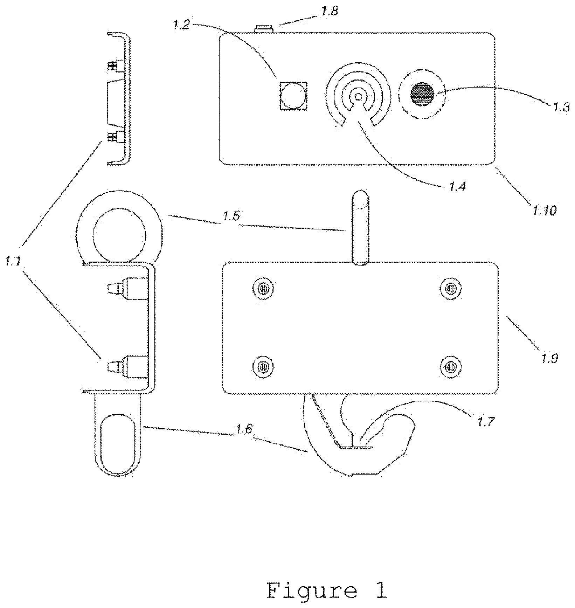

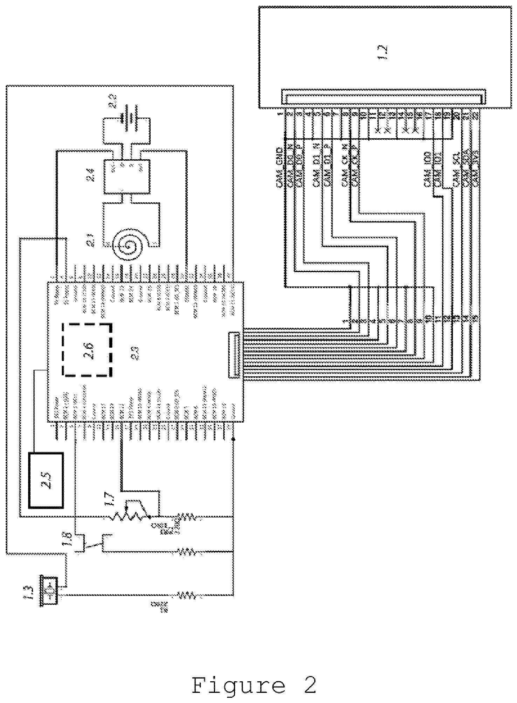

The multifunctional measuring device (Figure 1) has a rectangular shape capable of housing and enabling the use of a series of electronic circuits and sensors required for its purpose (Figure 2). As referred to in Figure 2, the device consists of a battery (2.2), an inductive charging module (2.1) for recharging the battery (2.2), a microprocessor (2.3), a charging control module (2.4), a pressure sensor (1.7), a push button (1.8), a buzzer (1.3) and a code reader (1.2), and is a multifunctional wireless control device, thus also comprising a wireless data transmission module (2.5) to a centralised monitoring module, not depicted. As mentioned in the general description, the code reader (1.2) performs the reading of the reference code, and this information is encrypted and sent to a centralised monitoring module, not depicted. The information sent to the centralised monitoring module may be encrypted, whereby the multifunctional device may comprise a cryptographic module (2.6) associated with the code reader and with the transmission module (2.5).

As shown in Figure 1, the components will be positioned separately and parallel, anchored on their corresponding pressure pins (1.1) to each portion (1.9. 1.10) of a housing.

On the front flat portion (1.10), there is the code reader (1.2), the buzzer (1.3) and a heat dissipater (1.4).

Another concave rectangular portion (1.9) contains on its upper side a closed upper hook (1.5), which is hung on the infusion support and should allow convenient handling, being sturdy and similar in appearance to a hanging ring of an infusion bottle. Its shape is compatible with all conventional approved intravenous infusion bottle holders.

On the lower side there is a specially designed, semi-open lower hook (1.6), which is larger than the upper hook (1.5). It has an integrated pressure sensor (1.7) that will measure the weight of the bottle. This sensor will give us all the required information to monitor each infusion process, in an individualised and automated way, in the chosen time pulses. This sensor is located in a depressed area in the lower hook (1.6), so as to ensure the position and integrity of the infusion bottle to the multifunctional device of the invention.

Since it is a gravity drip, the device has been designed to minimize its dimensions, avoiding possible interference due to loss of height from the infusion stand to the patient. Similarly, these infusion supports are usually adjustable in height, although we do not want to take this statement for granted, and therefore fit in the palm of the hand. It may have an anti-shock sheath.

These multifunctional devices will be recharged at a central charging base. Next to the charging base is the centralised monitoring module that will display the relevant data of those in use and will contain one or more touch screens for interaction with the system. It will also enable the implementation of a decentralised control application for mobile devices.

Intravenous infusion bottles are used daily in our medical centres and have a serious impact on the workload of our healthcare technicians and, as a result, their efficiency, performance and even their health are diminished. We have just lived through a global pandemic where it has been proven that it is necessary to minimise patient-nurse contact, and the invention in question facilitates the latter. It also involves a mental burden of control, which entails keeping a record of the usable ones in mind at all times. Added to that is the inclusion of QR codes for regulatory identification, which requires even more time-consuming data processing and attention for nurses. Said invention seeks, through the use of new technologies, a new methodology that encompasses an important part of the sub-processes included in the administration of intravenous usables (control of the usable, control of legitimacy, stock control, among others). At the same time, it improves the efficiency and work quality of our healthcare technicians.

The housing or body of the multifunctional device is formed almost entirely of plastic such as ABS or PLA+. It can also be covered with elastic anti-shock material. It may also contain metal parts, such as screws.

Rectangular in shape, it has two distinct parts: the flat part (1.10) that acts as a cover and at the same time contains certain features such as the code reader (1.2), the buzzer (1.3), the heat dissipater (1.4) and the push button (1.8), and another concave rectangular portion (1.9) that contains the microprocessors (2.3), not depicted in Figure 1, and integrated support modules (such as the inductive charging module (2.1), the charging control module (2.4), the data transmission module (2.5) and the cryptographic module (2.6)) also not depicted in Fig. 1) parallel in order, anchored to the pressure pins (1.1) and separated from each other to improve the performance and safety of the device.

This last concave rectangular portion (1.9) of the housing, of larger dimensions, can contain the hooks (1.5, 1.6). The upper hook (1.5) can be closed and rigid. The lower hook (1.6) can be open and rigid. It contains the pressure sensor (1.7) inside.

It also offers a roughness, not depicted, for ergonomic improvement, located opposite to the pushbutton (1.8).

## Claims

1. A multifunctional device for controlling intravenous drip infusion, of the type comprising means for measuring the amount of liquid remaining in an individual drip infusion unit, and which are associated with a centralised monitoring module via a wireless data transmission module (2.5); **characterised in that** it comprises a housing in which are arranged:
- an upper hook (1.5),
- a semi-open lower hook (1.6) in which a pressure sensor (1.7) is integrated,
- a graphic code reader (1.2) for capturing a reference code and sending it to the centralised monitoring module,
- a push button (1.8), associated with the microprocessor (2.3) and the code reader (1.2) to connect the use of said code reader (1.2),
- a battery (2.2), and
- a microprocessor (2.3).

2. The multifunctional device for controlling intravenous drip infusion according to claim 1, **comprising** a heat dissipater (1.4) arranged in the housing.

3. The multifunctional device for controlling intravenous drip infusion according to any of the preceding claims, **wherein** the pressure sensor (1.7) is located in a depressed area of the lower hook (1.6).

4. The multifunctional device for controlling intravenous drip infusion according to any of the preceding claims, **wherein** the lower hook (1.6) is larger than the upper hook (1.5).

5. The multifunctional device for controlling intravenous drip infusion according to any of the preceding claims, **comprising** a buzzer (1.3).

6. The multifunctional device for controlling intravenous drip infusion according to any of the preceding claims, **comprising** an inductive charging module (2.1) and a charging control module (2.4) connected to said inductive charging module (2.1), for recharging the battery (2.2).

7. The multifunctional device for controlling intravenous drip infusion according to any of the preceding claims, **comprising** a cryptographic module (2.6) associated with the code reader and the transmission module (2.5) for encrypting the information captured by the code reader (1.2) to be sent to the centralised monitoring module.

8. The multifunctional device for controlling intravenous drip infusion according to any of claims 5 to 7, **wherein** the housing comprises two differentiated portions:
- a flat portion (1.10), to which the code reader (1.2), the buzzer (1.3), the heat dissipater (1.4) and the push button (1.8) are attached, and
- another concave rectangular portion (1.9) inside of which the microprocessors (2.3) and support modules are located.

9. The multifunctional device for controlling intravenous drip infusion according to claim 8, **wherein** the microprocessors (2.3), and support modules located in the concave rectangular portion (1.9) of the housing are arranged in parallel and anchored to pressure pins (1.1).

10. The multifunctional device for controlling intravenous drip infusion according to claim 8 or 9, **wherein** the hooks (1.5, 1.6) are arranged in the concave rectangular portion (1.9) of the housing.

11. The multifunctional device for controlling intravenous drip infusion according to any of the preceding claims, **wherein** the housing has a roughness located opposite to the push button.

12. The multifunctional device for controlling intravenous drip infusion according to any of the preceding claims, **comprising** an anti-shock sheath.
